# EUROPÄISCHE PATENTSCHRIFT

(11) **EP 2 535 026 B1**
(45) Veröffentlichungstag und Bekanntmachung des Hinweises auf die Patenterteilung: **27.08.2014**
(21) Anmeldenummer: 12004477.1
(22) Anmeldetag: 13.06.2012
(51) Int. Cl.: D04H 1/541, D04H 1/558, D04H 1/70, A61F 13/00, B32B 3/28, B32B 5/26, B32B 3/12, B32B 5/02

(54) **Verband**
Bandage
Bandage

(30) Priorität: 15.06.2011 DE 102011106495
(43) Veröffentlichungstag der Anmeldung: 19.12.2012
(73) Patentinhaber: Östreicher, Ulrich, 37276 Meinhard (DE)
(72) Erfinder: Östreicher, Ulrich, 37276 Meinhard (DE)
(74) Vertreter: Gittinger, Andreas

(56) Entgegenhaltungen:
- EP-A1- 0 045 592
- EP-A1- 1 543 803
- EP-A1- 1 884 253
- WO-A1-2010/052485
- DE-A1- 2 820 793
- DE-A1-102004 031 955
- DE-A1-102006 050 806
- DE-U1- 29 821 466
- DE-U1-202007 014 618
- DE-U1-202011 001 797

## Beschreibung

Die Erfindung betrifft einen Verband, insbesondere einen Stütz-, Fixier- oder Druckverband gemäß dem Oberbegriff des Anspruchs 1.

Die aus dem Stand der Technik bekannten Verbände haben den Nachteil, dass sie aus der Umgebung stammende Bakterien ungehindert an eine unter dem Verband befindliche Wunde gelangen lassen.

Die EP 0 045 592 A1 beschreibt einen Verband mit einer eine Rückstellkraft erzeugenden zentralen Spannlage. An der Spannlage wird oben und unten jeweils ein Vlies aufkalandriert. Die Spannlage kann jedoch Makro- und Mikroporen aufweisen. Somit ist eine zuverlässige Barriere gegen Bakterien ebenfalls nicht zuverlässig gewährleistet.

Die Erfindung hat die Aufgabe, den gattungsgemäßen Verband dahingehend weiterzuentwickeln, dass der oben genannte Nachteil zukünftig vermieden wird.

Die Erfindung löst die gestellte Aufgabe mit einem Verband gemäß Anspruch 1. Somit ist die Gefahr, dass aus der Umgebung stammende Bakterien an eine unter dem Verband befindliche Wunde gelangen können, deutlich reduziert. Dadurch wird die Gefahr einer Infektion deutlich reduziert und ein Wundheilungsprozess gefördert.

Um zuverlässig verhindern zu können, dass Bakterien aus der Umgebung durch den Verband an die Wunde gelangen können, ist die Barrierelage einseitig mit einer den Durchgang der Bakterien blockierenden Barriereschicht beschichtet. Durch die einseitige Beschichtung lässt sich die Barrierelage preiswert herstellen, insbesondere dann, wenn die Barrierelage als eine Folie ausgeführt ist und die einseitige Beschichtung somit auf einer Seite der als Barrierelage dienenden Folie aufgebracht ist. Vorzugsweise ist die Barriereschicht auf der der Wunde abgewandten Seite vorgesehen. Grundsätzlich ist es jedoch möglich, dass sie auf der der Wunde zugewandten Seite aufgebracht ist.

Damit trotz des gehinderten Durchgangs der Bakterien, ein Luftaustausch durch den Verband stattfinden kann, kann die Barrierelage wasserdampfdurchlässig sein.

Das mindestens eine Vlies kann eine eine Formstabilität erhöhende Stabilisierungslage sein. Somit werden unerwünschte Einschnürungen des Verbandes deutlich reduziert, wodurch der Tragekomfort des Verbandes verbessert wird.

In der Praxis erweist es sich als besonders vorteilhaft, dass die Barrierelage zwischen der mindestens einen Spannlage und dem mindestens einen Vlies angeordnet ist.

Zu beiden Seiten der mindestens einen Spannlage kann jeweils mindestens ein Vlies angeordnet sein. Da das Vlies vorzugsweise eine Feuchtigkeit aufnehmende Wirkung hat, kann der Verband besonders effektiv die Feuchtigkeit aufnehmen, wenn zu beiden Seiten der Spannlage mindestens ein Vlies vorgesehen ist.

Damit der Verband beim Anlegen gedehnt werden kann, kann die Barrierelage und/oder das mindestens eine Vlies wellenartig ausgebildet sein. Aus demselben Grund kann die Spannlage aus einem elastischen, eine hohe Rückstellkraft aufweisenden Material hergestellt sein.

In einer bevorzugten Ausführungsform können die Barrierelage und das als Stabilisierungslage dienende Vlies vollflächig aneinander anliegen. Wenn die Barrierelage als Folie ausgeführt ist, weist die Barrierelage von Natur aus keine Formstabilität auf. Durch die vollflächige Anlage der Folie an der die Formstabilität aufweisenden Stabilisierungslage erhält die Folie jedoch eine deutlich stabilere Form. Die Barrierelage respektive die Folie kann deshalb noch dünner ausgeführt werden, wodurch die Wasserdampfdurchlässigkeit verbessert wird. Außerdem weist der Verband somit eine geringere Dicke auf und kann preisgünstiger produziert werden.

Die mindestens eine Spannlage, die Barrierelage und das mindestens eine Vlies können punktuell miteinander verbunden sein, so dass der Verband mehrere Lagen aufweisen kann, die dem Verband einerseits eine bestimmte Formstabilität verleihen und ihm andererseits eine bestimmte Dehnbarkeit verleihen. Die punktuelle Verbindung kann vorzugsweise durch Kleben oder Schweißen hergestellt werden.

Die mindestens eine Spannlage kann eine Wabenstruktur aufweisen. Die Wabenstruktur verleiht dem Verband eine gewisse Dicke, so dass seine Formstabilität erhöht wird. Vorzugsweise erstreckt sich die Tiefe von die Wabenstruktur bildenden Waben quer zur Längsrichtung und in Richtung der Breite des Verbandes. Grundsätzlich ist es jedoch auch möglich, dass sich die Tiefe der Waben quer zur Längsrichtung aber in Richtung der Dicke des Verbandes erstreckt.

Das mindestens eine Vlies kann ein Spinnvlies sein, welches besonders wirksam die Feuchtigkeit aufnehmen kann. Nachfolgend wird ein Ausführungsbeispiel eines erfindungsgemäßen Verbandes anhand der beiliegenden Zeichnungen näher erläutert.

Im Einzelnen zeigen:
- Fig. 1: einen schematischen Längsschnitt durch eine erste Ausführungsform des Verbandes;
- Fig. 2: eine Wabenstruktur.

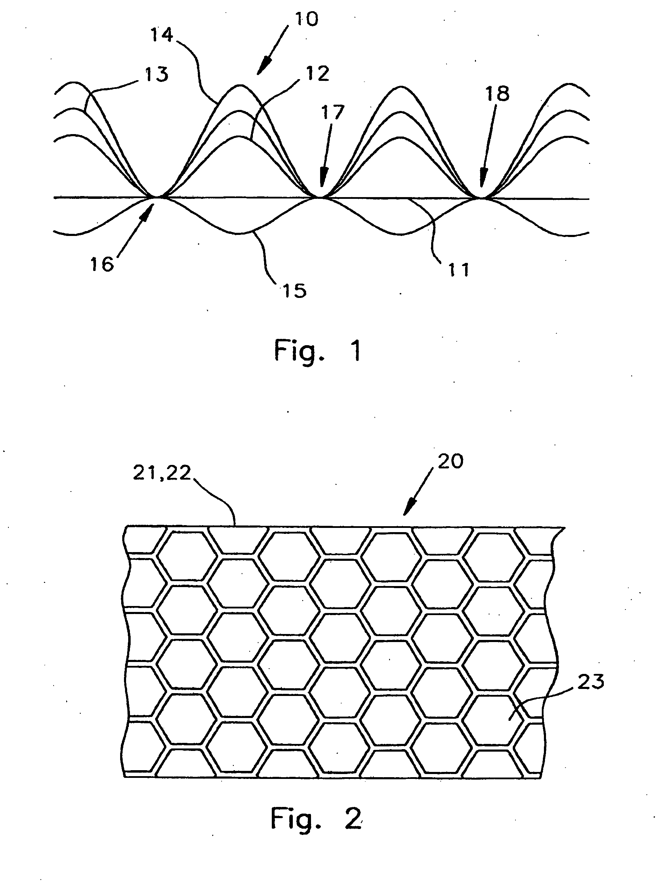

Fig. 1 zeigt einen Verband 10, der ein Stütz-, Fixier- oder Druckverband sein kann. Im mittleren Bereich ist eine eine Rückstellkraft erzeugende Spannlage 11 angeordnet. Die Spannlage 11 ist aus einem elastischen Material hergestellt, das eine hohe Rückstellkraft aufweist. Der Verband kann somit gedehnt werden und weist folglich eine hohe Anpressung auf einen zu verbindenden Körperbereich, insbesondere auf eine Wunde eines Patienten auf.

Unmittelbar angrenzend an die Spannlage 11 ist eine Barrierelage 12 angeordnet, die vorzugsweise als eine Folie ausgeführt sein kann. Die Barrierelage 12 verhindert einen Durchgang von aus der Umgebung stammenden Bakterien zu einer verbundenen Wunde hin. Die Barrierelage 12 ist mit einer den Durchgang der Bakterien blockierenden, aber hier nicht näher dargestellten Barriereschicht auf einer Seite versehen. Die Barrierelage 12 ist wasserdampfdurchlässig, so dass ein Luftaustausch durch den Verband 10 möglich ist. Folglich können auch Ausdünstungen vom Körper des Patienten an die Umgebung gelangen.

Unmittelbar angrenzend an die Barrierelage 12 ist ein Vlies 13 angeordnet. Das Vlies 13 ist eine eine Formstabilität des Verbandes 10 erhöhende Stabilisierungslage. Sie reduziert unerwünschte Einschnürungen des Verbandes 10 und erhöht somit den Tragekomfort für den Patienten.

Der Verband 10 weist an seinen beiden Außenseiten ein Vlies 14 und 15 auf. Die Vliese 14 und 15 können Feuchtigkeit aufnehmen und schützen auch die Haut des Patienten. Grundsätzlich können die Vliese 14 und 15 jedoch auch wasserabstoßend ausgerüstet sein. Die Vliese 14 und 15 können vorzugsweise mittels eines Wasserstrahlverfahrens hergestellt werden, so dass sie besonders weich und zugleich reißfest sind.

Die Vliese 13, 14 und 15 und die Barrierelage 12 sind wellenartig ausgebildet. Somit kann der Verband 10 in seiner Längsrichtung gedehnt werden und auch durch die die Rückstellkraft aufweisende Spannlage 11 wenigstens teilweise wieder verkürzt werden.

Die Spannlage 11, die Barrierelage 12, die Vliese 13, 14 und 15 sind an Verbindungsstellen 16, 17 und 18 punktuell miteinander verbunden, wobei die punktuelle Verbindung vorzugsweise durch Kleben oder Schweißen hergestellt wird. An den Verbindungsstellen 16, 17 und 18 stehen lediglich unmittelbar benachbarte Lagen miteinander in Verbindung. Folglich ist die Spannlage 11 mit der Barrierelage 12 und dem Vlies 15 unmittelbar verbunden. Ebenso ist das als Stabilisierungslage dienende Vlies 13 unmittelbar mit der Barrierelage 12 und dem Vlies 14 unmittelbar verbunden. Aus Gründen der besseren Erkennbarkeit sind die Barrierelage 12 und die Stabilisierungslage 13 als voneinander getrennt dargestellt. Eine solche Anordnung der Barrierelage 12 und der Stabilisierungslage 13 ist zwar möglich, vorzugsweise liegen die Barrierelage 12 und die Stabilisierungslage 13 jedoch vollflächig aneinander an. Dies ist besonders dann sinnvoll, wenn die Barrierelage 12 als Folie ausgeführt ist.

Fig. 2 zeigt eine Wabenstruktur 20 einer Spannlage 21 oder eines Vlieses 22. Die Tiefe von Waben 23 der Wabenstruktur 20 kann sich vorzugsweise quer zur Längsrichtung und in Richtung der Breite der Spannlage 21 oder des Vlieses 22 erstrecken. Es ist jedoch auch möglich, dass sich die Tiefe der Waben 23 quer zur Längsrichtung und in Richtung der Dicke der Spannlage 21 oder des Vlieses 22 erstrecken.

### BEZUGSZEICHENLISTE

- 10: Verband
- 11: Spannlage
- 12: Barrierelage
- 13: Vlies
- 14: Vlies
- 15: Vlies
- 16: Verbindungsstelle
- 17: Verbindungsstelle
- 18: Verbindungsstelle

- 20: Wabenstruktur
- 21: Spannlage
- 22: Vlies
- 23: Wabe

## Patentansprüche

1. Verband (10), insbesondere Stütz-, Fixier- oder Druckverband mit mindestens einer eine Rückstellkraft erzeugenden Spannlage (11), mindestens einem Vlies (13, 14, 15) und einer einen Durchgang von Bakterien blockierende Barrierelage (12), **dadurch gekennzeichnet, dass** die Barrierelage (12) einseitig mit einer den Durchgang der Bakterien blockierenden Barriereschicht beschichtet ist und die Barrierelage (12) zwischen der mindestens einen Spannlage (11) und dem mindestens einen Vlies (13, 14) angeordnet ist.

2. Verband (10) nach Anspruch 1, **dadurch gekennzeichnet, dass** die Barrierelage (12) wasserdampfdurchlässig ist.

3. Verband (10) nach Anspruch 1 oder 2, **dadurch gekennzeichnet, dass** das mindestens eine Vlies (13) ein eine Formstabilität erhöhende Stabilisierungslage ist.

4. Verband (10) nach einem der Ansprüche 1 bis 3, **dadurch gekennzeichnet, dass** zu beiden Seiten der mindestens einen Spannlage (11) jeweils mindestens ein Vlies (13, 14, 15) angeordnet ist.

5. Verband (10) nach einem der Ansprüche 1 bis 4, **dadurch gekennzeichnet, dass** die Barrierelage (12) und/oder das mindestens eine Vlies (13, 14, 15) wellenartig ausgebildet ist.

6. Verband (10) nach einem der Ansprüche 3 bis 5, **dadurch gekennzeichnet, dass** die Barrierelage (12) und das als Stabilisierungslage dienende Vlies (13) vollflächig aneinander anliegen.

7. Verband (10) nach einem der Ansprüche 1 bis 6, **dadurch gekennzeichnet, dass** die mindestens eine Spannlage (11), die Barrierelage (12) und das mindestens eine Vlies (13, 14, 15) punktuell miteinander verbunden sind.

8. Verband (10) nach einem der Ansprüche 1 bis 7, **dadurch gekennzeichnet, dass** die mindestens eine Spannlage (11) eine Wabenstruktur aufweist.

9. Verband (10) nach einem der Ansprüche 1 bis 8, **dadurch gekennzeichnet, dass** das mindestens eine Vlies (13, 14, 15) ein Spinnvlies ist.

## Claims

1. A dressing (10), more particularly support, fixation or compression dressing with at least one tensioning layer (11), which produces a restoring force, at least one non-woven material (13, 14, 15) and a barrier layer (12) that blocks the passage of bacteria, **characterised in that** on one side the barrier layer (12) is coated with a barrier coating that blocks the passage of bacteria and the barrier layer (12) is arranged between the at least one tensioning layer (11) and the at least one non-woven material (13, 14).

2. The dressing (10) according to claim 1, **characterised in that** the barrier layer (12) is impermeable to water vapour.

3. The dressing (10) according to claim 1 or 2, **characterised in that** the at least one non-woven material (13) is a stabilisation layer that increases the dimensional stability.

4. The dressing (10) according to any one of claims 1 to 3, **characterised in that** arranged on both sides of the at least one tension layer (11) there is at least one non-woven material (13, 14, 15).

5. The dressing (10) according to any one of claims 1 to 4, **characterised in that** the barrier layer (12) and/or the at least one non-woven material (13, 14, 15) is undulating in shape.

6. The dressing (10) according to any one of claims 3 to 5, **characterised in that** the barrier layer (12) and the non-woven material (13) serving as a stabilisation layer are in contact with each other over their entire surface.

7. The dressing (10) according to any one of claims 1 to 6, **characterised in that** the at least one tensioning layer (11), the barrier layer (12) and the at least one non-woven material (13, 14, 15) are connected to each other in a point-wise manner.

8. The dressing (10) according to any one of claims 1 to 7, **characterised in that** the at least one tensioning layer (11) has a honeycomb structure.

9. The dressing (10) according to any one of claims 1 to 8, **characterised in that** the at least one non-woven material (13, 14, 15) is a spunbond material.

## Revendications

1. Assemblage, en particulier assemblage de soutien, fixation ou de compression, comportant au moins une couche de serrage (11) générant une force de rappel, au moins un non-tissé (13, 14, 15) et une couche formant barrière (12) bloquant un passage de bactéries, **caractérisé en ce que** la couche formant barrière (12) est revêtue d'un côté d'une épaisseur formant barrière bloquant le passage des bactéries et que la couche formant barrière (12) est disposée entre l'au moins une couche de serrage (11) et l'au moins un non-tissé (13, 14).

2. Assemblage selon la revendication 1, **caractérisé en ce que** la couche formant barrière (12) est perméable à la vapeur d'eau.

3. Assemblage selon la revendication 1 ou 2, **caractérisé en ce que** l'au moins un non-tissé (13) est une couche stabilisatrice augmentant la stabilité de la forme.

4. Assemblage selon une des revendications 1 à 3, **caractérisé en ce que**, des deux côtés de l'au moins une couche de serrage (11), respectivement un non-. tissé (13, 14, 15) est disposé.

5. Assemblage selon une des revendications 1 à 4, **caractérisé en ce que** la couche formant barrière (12) et/ou l'au moins un non-tissé (13, 14, 15) ont une conformation ondulée.

6. Assemblage selon une des revendications 3 à 5, **caractérisé en ce que** la couche formant barrière (12) et le non-tissé (13) servant de couche stabilisatrice sont en contact mutuel par toute leur surface.

7. Assemblage selon une des revendications 1 à 6, **caractérisé en ce que** l'au moins une couche de serrage (11), la couche formant barrière (12) et l'au moins un non-tissé (13, 14, 15) sont raccordés ponctuellement entre eux.

8. Assemblage selon une des revendications 1 à 7, **caractérisé en ce que** l'au moins une couche de serrage (11) présente une structure en nid d'abeille.

9. Assemblage selon une des revendications 1 à 8, **caractérisé en ce que** l'au moins un non-tissé (13, 14, 15) est un filé-lié.
